# EUROPEAN PATENT SPECIFICATION

(11) **EP 3 017 833 B1**
(45) Date of publication and mention of the grant of the patent: **02.05.2018**
(21) Application number: 15192296.0
(22) Date of filing: 30.10.2015
(51) Int. Cl.: A61M 1/00

(54) **SUCTION CONTROL APPARATUS FOR USE IN LAPAROSCOPY**
SAUGSTEUERUNGSVORRICHTUNG ZUR VERWENDUNG IN DER LAPAROSKOPIE
APPAREIL DE COMMANDE D'ASPIRATION DESTINÉ À ÊTRE UTILISÉ DANS UNE LAPAROSCOPIE

(30) Priority: 10.11.2014 GB 201419943; 27.11.2014 GB 201421086
(43) Date of publication of application: 11.05.2016
(73) Proprietor: VacSax Limited, Plymoutn, Devon PL7 5BG (GB)
(72) Inventor: Bennett, John, Plymouth, Devon PL7 5BG (GB)
(74) Representative: Bryers LLP

(56) References cited:
- US-A1- 2014 058 328

## Description

### Field of the Invention

The present invention relates to suction control apparatus, in particular to controlling suction flow rate during laparoscopic surgery.

### Background of the Invention

Laparoscopy is a type of surgical procedure in which investigations or operations are performed within the abdominal or pelvic cavity using a laparoscope inserted into the body through a small incision made in the skin, typically near the navel. The laparoscope has a camera and light, and allows internal structures to be seen clearly on an external visual display screen. Laparoscopic surgery, which is also known as keyhole surgery or minimally invasive surgery, allows a surgeon to access and view the inside of the abdomen and pelvis of the body without needing to make large incisions in the skin. In addition to the laparoscope, tubes, probes, small surgical instruments and suction and irrigation sets can be introduced into the body as required using the same or other small incisions.
During a laparoscopy procedure, the abdomen is inflated with a gas in order to obtain more easily intelligible images from the laparoscope and also to increase the room inside the abdominal and pelvic cavities in which the surgeon can work. During the course of surgery, fluid is pumped into the abdomen to clean the surgical site and suction is used to remove this fluid along with any other bodily fluids and tissue.

Hand-held laparoscopic suction and irrigation sets are known that comprise two separate channels, which, in use, are connected one to a suction unit and the other to an irrigation system, and a handle housing a valve arrangement and provided with buttons to allow a surgeon to control suction and irrigation functions. With this system, suction has to be left turned on downstream of the handle throughout the procedure.

Historically, hospital central suction systems, to which a hand-held laparoscopic suction and irrigation is typically connected, are designed for providing relatively high levels of suction over relatively short periods, and are not designed for providing maintained levels of suction for long periods of time. It is known for a hospital central suction system to provide suction at a generated vacuum pressure level as high as 750 mmHg.

Often, laparoscopic suction and irrigation sets are operated using the maximum vacuum pressure level available from the hospital central suction system. Consequently, suction collection canisters, which are used to store fluids removed from a patient during surgery and which are usually made from a plastics material, are subject to excessive long term applied stress. Over time, there develops a considerable risk of implosion of the suction collection canisters. Clearly, it is desirable to prevent failure of the suction collection canisters to safely contain collected fluids.

In addition, in existing systems, when the surgeon demands suction by operating the relevant button on the laparoscopic suction and irrigation set handle, a high suction flow rate is immediately generated under a high vacuum pressure level from the hospital central suction system. The laparoscopic suction and irrigation set does not provide the surgeon with any control over the suction flow rate. Consequently, if the flow rate under suction exceeds the flow rate of medical gas being pumped into the abdominal cavity, the abdomen will start to collapse. This not only has the effect of restricting the surgeon's view of the surgical site, but also limits the length of time the surgeon can use suction and necessitates a period of resting to allow for reinflation of the abdominal cavity.

Currently, in the UK at least, there are no working guidelines concerning maximum safe levels of vacuum pressure and suction flow rate during surgical procedures.

During surgery, safe suctioning is achievable using a relatively high suction flow rate at a relatively low vacuum pressure level. However, the suction flow rate produced in the laparoscopic suction and irrigation set by the suction system is affected by multiple factors, including the degree of differential pressure created under the operation of the suction system, the viscosity of the matter being suctioned, and also the flow resistance due to features of the apparatus such as tubing, Therefore, the vacuum pressure level required to achieve a particular suction flow rate can vary between different laparoscopic suction and irrigation sets. From a different perspective, the suction flow rate produced within different laparoscopic suction and irrigation sets operated using the same suction system and the same vacuum pressure level can vary. It is further to be appreciated that the flow of fluid during suctioning may be laminar or turbulent, with this aspect also affecting the relationship between vacuum pressure level and suction flow rate.
US Patent Publication No. US 2014/0058328 A1 discloses a system and method to vent gas from a body cavity during an endoscopic procedure, in which a vacuum break device has a chamber in fluid communication with an exhaust gas inlet and an exhaust gas outlet, the chamber comprises one or more openings in fluid communication with the atmosphere, a body cavity is in fluid communication with the exhaust gas inlet and the exhaust gas outlet is connected directly or indirectly to a suction source.

### Summary of the Invention

The invention is defined in the appended claims.

According to a first aspect there is provided suction control apparatus for controlling suction flow rate during laparoscopic surgery, comprising: a suction flow path between an inlet and an outlet, said inlet connectable to a suction collection system comprising at least one fluid collection canister, and said outlet connectable to a suction-generating vacuum source, said suction-generating vacuum source, when connected to said outlet, operable to generate a vacuum source vacuum pressure level that produces a vacuum source suction flow rate within said suction flow path; and a user-adjustable suction flow rate controller operable to control the suction flow rate in said suction flow path; a vacuum pressure level regulator operable to regulate the vacuum pressure level within said suction flow path to prevent a pre-set, user non-adjustable regulated maximum suction vacuum pressure level being exceeded.

In an embodiment, the suction flow rate controller comprises a flow control valve.

In an embodiment, the suction flow rate is adjustable within a range having a pre-set user non-adjustable lower limit and a pre-set user non-adjustable upper limit.

In an embodiment, the suction control apparatus may further comprise a user-operable over-ride valve operable to bypass the vacuum regulator and the suction flow rate controller.

In an embodiment, the suction control apparatus further comprises a flow rate indicator for indicating the suction flow rate within the suction flow path.

In an embodiment, the suction control apparatus further comprises a vacuum pressure gauge for indicating the vacuum pressure level within the suction flow path.

In an embodiment, the suction control apparatus is housed by a suction control unit. The suction control unit may then be mountable to a trolley for supporting at least one fluid collection canister.

In an embodiment, the pre-set, user non-adjustable maximum regulated vacuum pressure level is in the range between a lower limit of equal to or greater than 250 mmHg and an upper limit of less than or equal to 350 mmHg

In an embodiment, the suction flow rate is adjustable within the range between a lower limit equal to or greater than 10 litres per minute and an upper limit less than or equal to 100 litres per minute.

The outlet of the suction control apparatus may be connectable to a hospital central vacuum system or a portable electric suction unit.

According to a second aspect there is provided apparatus for use in performing laparoscopy, comprising suction control apparatus according to the first aspect.

Different aspects and embodiments of the invention may be used separately or together.

Further particular and preferred aspects of the present invention are set out in the accompanying independent and dependent claims. Features of the dependent claims may be combined with the features of the independent claims as appropriate, and in combination other than those explicitly set out in the claims, without departing from the scope of the invention as defined by the appended claims.

### Brief Description of the Drawings

The present invention will now be more particularly described, with reference to the accompanying drawings, in which:
**Figure 1** shows a diagram of laparoscopic suction and irrigation apparatus comprising suction control apparatus according to the present invention;
**Figure 2** shows a schematic of the suction control apparatus;
**Figure 3** shows a suction control unit housing the suction control apparatus; and
**Figure 4** shows the suction control unit mounted to a trolley.

### Description

Example embodiments are described below in sufficient detail to enable those of ordinary skill in the art to embody and implement the apparatus, systems and processes herein described. It is important to understand that embodiments can be provided in many alternate forms and should not be construed as limited to the examples set forth herein and that the scope of the invention is defined by the appended claims.

The present invention provides a safe suction system with flow rate control, for use by a surgeon during laparoscopic surgery.

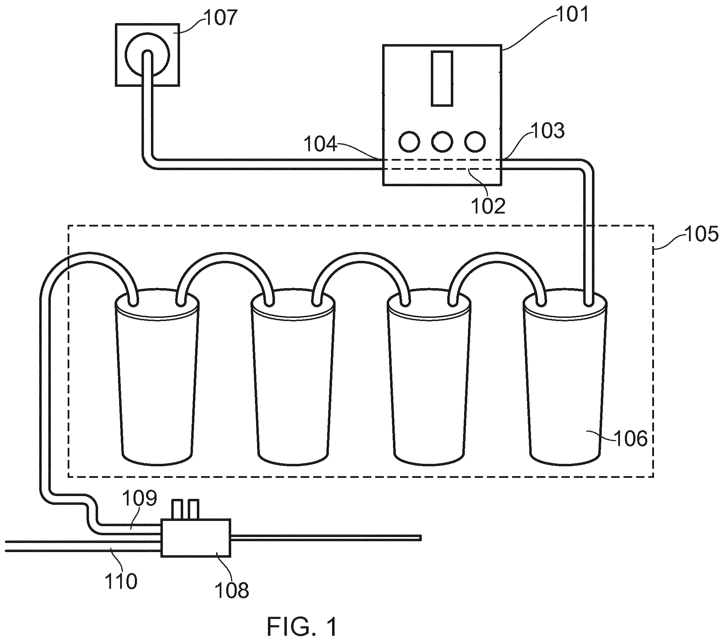

A diagram of laparoscopic suction and irrigation apparatus comprising suction control apparatus 101 according to an embodiment of the present invention is shown in **Figure 1****.** The suction control apparatus 101 comprises a suction flow path, indicated at 102, between an inlet 103 and an outlet 104. The inlet 103 is connectable to a suction collection arrangement, indicated at 105, comprising at least one fluid collection canister 106. In the example arrangement shown in this Figure, the suction collection arrangement comprises a series of four collection canisters, which have a plastics material-based fabrication. The outlet 104 is connectable to a suction-generating vacuum source 107. In the example arrangement shown in this Figure, the suction-generating vacuum source 107 is a hospital central suction system port. A laparoscopic suction and irrigation set 108 is also shown. The laparoscopic suction and irrigation set 108 has a suction channel 109 connectable to the suction collection arrangement 105, and hence connectable via the suction collection arrangement 105 to the inlet 103 of the suction control apparatus 101, and an irrigation channel 110 connectable to an irrigation system. When the suction-generating vacuum source 107 is connected to the outlet 104 of the suction control apparatus 101, the suction-generating vacuum source 107 is operable to generate a vacuum source vacuum level that produces a vacuum source suction flow rate within the suction flow path 102.

In an application, the suction-generating vacuum source 107, to which the outlet 104 of the suction control apparatus 101 is connected, is a portable electric suction unit.

As mentioned above, in an application, the suction-generating vacuum source 107, to which the outlet 104 of the suction control apparatus 101 is connected, is a hospital central suction system.

It is known for a hospital central suction system to provide suction at a generated vacuum source vacuum pressure level as high as 750 mmHg, and problems related to this high level of vacuum pressure have been experienced by surgeons performing laparoscopic procedures.

It is found that fluid collection canisters are subject to excessive long term applied stress under prolonged high suction levels, which can lead to the fluid collection canisters imploding or failing. The risk of collected fluids not being properly retained is an undesirable health and safety hazard. In addition, it has been found that the suction flow rate produced under such a high vacuum pressure level can be greater than the flow rate of medical gas being pumped into the abdominal cavity, causing the abdomen to deflate. Consequently, internal images displayed to the surgeon are impaired and progress is hindered as the length of time the surgeon can use suction is constrained and periods of rest time are required to allow for reinflation of the abdominal cavity.

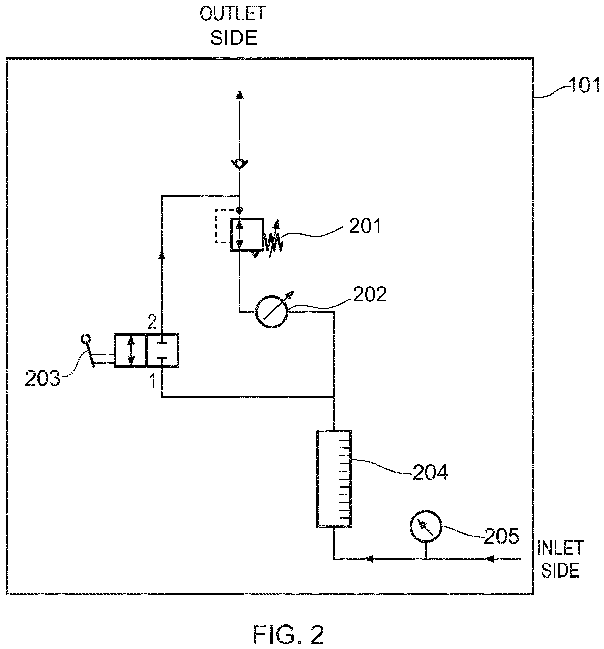

**Figure 2** shows a schematic of the suction control apparatus 101. The suction control apparatus 101 comprises a vacuum pressure level regulator 201 operable to regulate the vacuum pressure level within the suction flow path between the inlet and the outlet to a pre-set, user non-adjustable maximum regulated vacuum pressure level. The vacuum pressure level regulator 201 is advantageously operable to reduce the vacuum pressure level within the suction flow path to a regulated maximum suction vacuum pressure level that is less than the vacuum source vacuum pressure level produced in the suction flow path by the suction-generating vacuum source to which the suction control apparatus 101 is connected.

Thus, the vacuum pressure level regulator 201 of the suction control apparatus 101 can be factory set to a maximum regulated vacuum pressure level that has been predetermined to be a safe pressure vacuum level for surgical procedures and fluid collection canisters. This safe maximum regulated vacuum pressure level is user non-adjustable. It is to be appreciated that the pre-set maximum regulated vacuum pressure level to which the valve regulator 201 of the suction control apparatus 101 is factory set may vary between particular apparatus and intended applications.

In an embodiment, the pre-set, user non-adjustable maximum regulated vacuum level is in the range between a lower limit of equal to or greater than 250 mmHg and an upper limit of less than or equal to 350 mmHg. In an example, the pre-set, user non-adjustable maximum regulated vacuum level is, or is approximately, 300 mmHg.

The suction control apparatus 101 further comprises a user-adjustable suction flow rate controller 202 operable to control the suction flow rate in the suction flow path. In this illustrated embodiment, the suction flow rate controller 202 comprises a flow control valve. In an embodiment, the suction flow rate is adjustable within a range having a pre-set user non-adjustable lower limit and a pre-set user non-adjustable limit.

Thereby, the suction flow rate control valve 202 is beneficially operable to allow a surgeon to reduce or increase the suction flow rate, as desired, during surgery. Using this function, the surgeon can overcome issues with unwanted abdominal cavity deflation.

In an embodiment, the suction flow rate is adjustable in the range between a lower limit of equal to or greater than 10 litres per minute and an upper limit of less than or equal to 100 litres per minute. However, it is to be appreciated that the range within which the suction flow rate is adjustable may vary between particular apparatus and intended applications.

Preferably, and in this illustrated embodiment, the suction control apparatus 101 comprises an user-operable over-ride valve 203 operable to bypass the vacuum regulator 201 and the suction flow controller 202. Using the over-ride valve 203 the surgeon can selectively opt to obtain a short burst or boost of higher suction as required. In this way, potentially useful higher suction provided by the suction-generating vacuum source can be utilised on demand.

Preferably, and in this illustrated embodiment, the suction control apparatus 101 comprises a flow rate indicator 204 for indicating the suction flow rate within the suction flow path. In this way, a useful visual display of the suction flow rate is provided. This allows the surgeon to readily monitor the suction flow rate and conveniently use the display as a guide when making adjustments that affect the suction flow rate. The flow rate indicator 204 may take any suitable form, and may include such features as alphanumeric characters, symbols, colour coding, a dial, a digital display.

Preferably also, and in this illustrated embodiment, the suction control apparatus 101 comprises a vacuum pressure level gauge 205 for indicating the vacuum pressure level within the suction flow path. This provides a helpful visual indication as to the operational vacuum pressure level at any time. The vacuum pressure level gauge 205 may take any suitable form, and may include such features as alphanumeric characters, symbols, colour coding, a dial, a digital display.

It is to be appreciated that the suction control apparatus 101 may comprise further componentry or features.

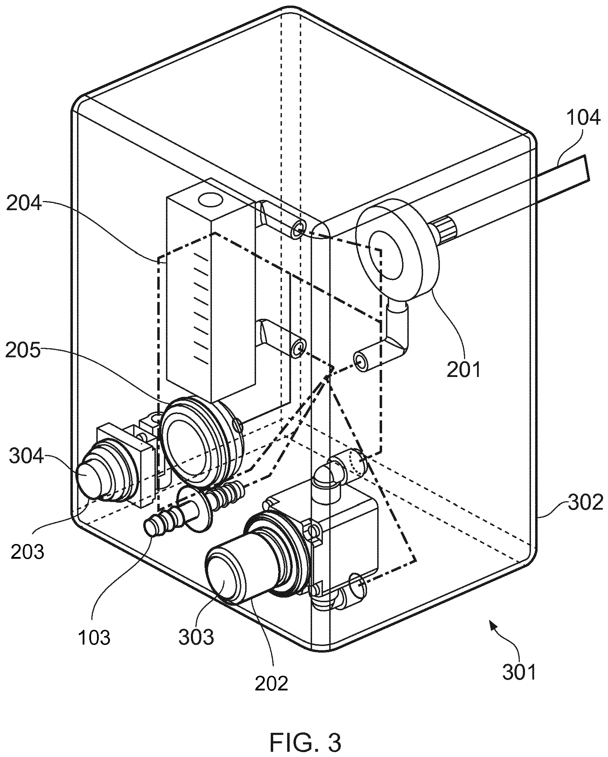

**Figure 3** shows the suction control apparatus housed by a suction control unit 301. The suction control unit 301 comprises a casing 302.

The inlet 103 and outlet 104 of the suction control apparatus may be provided with any suitable connection elements for allowing connection to a suction collection arrangement and suction-generating vacuum source respectively. In an example, a tubular element extends from the outlet 104 and it provided with a plug for connection to a socket associated with the suction-generating vacuum source.

A manually operable element 303 is provided to allow operation of the suction flow controller 202. In this embodiment, the manually operable element 303 is a dial. The suction flow controller 202 and manually operable element 303 may be arranged to allow selection of any suction flow rate within an available range of suction flow rate (continuous range) or may be arranged to allow selection of one of a plurality of discrete suction flow rates within an available range of suction flow rate (distinct value range).

A manually operable element 304 is provided to allow operation of the over-ride valve 203. In this embodiment, the manually operable element 304 is a push button. Preferably, and in this embodiment, the manually operable element 304 is a momentary push button such that the over-ride function operates for only as long as the push button is depressed.

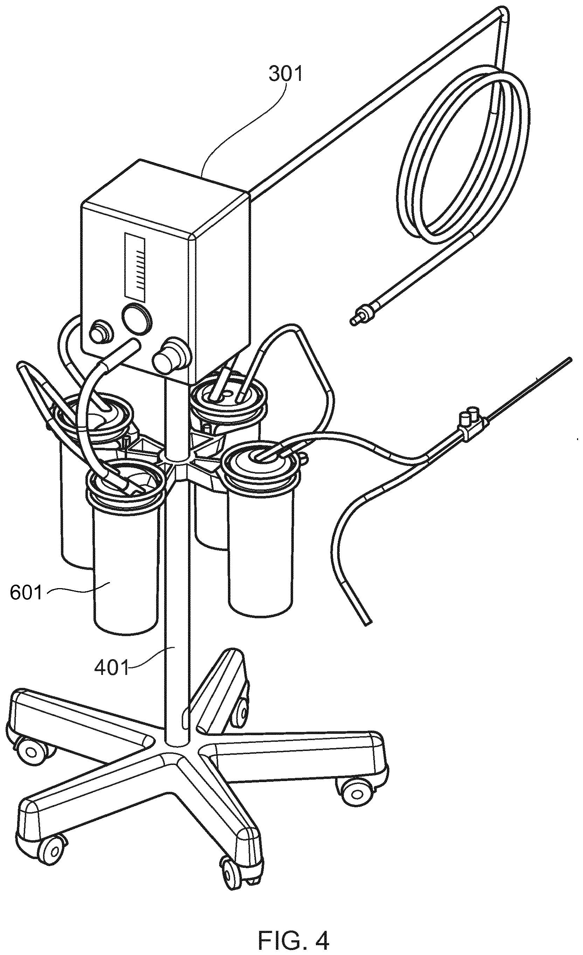

As shown in **Figure 4****,** the suction control unit 30I is mountable to a trolley 401 for supporting one or more fluid collection canisters, such as fluid collection canister 106.

The present invention thus provides suction control apparatus advantageously comprising a suction flow path with a pre-set, user non-adjustable maximum vacuum pressure level for regulating the maximum vacuum pressure level within the suction flow path and a user-adjustable suction flow controller for adjusting the suction flow rate within the suction flow path. The suction control apparatus of the present invention thus beneficially provides for suction to be performed at a vacuum pressure level not exceeding a predetermined maximum safe vacuum pressure level and at a selectable suction flow rate.

The suction control apparatus of the present invention is of particular applicability in the field of laparoscopy. Suction control apparatus as described herein, with reference to the accompanying drawings, is usable during laparoscopic surgery to limit the vacuum level to a maximum, safe vacuum level yet allow the suction flow rate to be adjusted within a range of suction flow rates.

The present invention provides suction control apparatus, in particular for controlling suction flow rate during laparoscopic surgery. The present invention also provides apparatus for use in performing laparoscopy that comprises the suction control apparatus. The present invention further provides a laparoscopic suction and flow control system.

Although illustrative embodiments of the invention have been disclosed in detail herein, with reference to the accompanying drawings, it is understood that the invention is not limited to the precise embodiments shown and that various changes and modifications can be effected therein by one skilled in the art without departing from the scope of the invention as defined by the appended claims.

## Claims

1. Suction control apparatus (101) for controlling suction flow rate during laparoscopic surgery, comprising:
a suction flow path (102) between an inlet (103) and an outlet (104),
said inlet (103) connectable to a suction collection system (105) comprising at least one fluid collection canister (106), and
said outlet (104) connectable to a suction-generating vacuum source (107), said suction-generating vacuum source (107) when connected to said outlet (104), operable to generate a vacuum source vacuum pressure level that produces a vacuum source suction flow rate within said suction flow path (102); and
a user-adjustable suction flow rate controller (202) operable to control the suction flow rate in said suction flow path (102); **characterised by**:
a vacuum pressure level regulator (201) operable to regulate the vacuum pressure level within said suction flow path (102) to prevent a pre-set, user non-adjustable regulated maximum suction vacuum pressure level being exceeded.

2. Suction control apparatus (101) as claimed in claim 1, wherein said user-adjustable suction flow rate controller (202) comprises a flow control valve.

3. Suction control apparatus (101) as claimed in claim 1 or claim 2, wherein said suction flow rate is adjustable within a range having a pre-set user non-adjustable lower limit and a pre-set user non-adjustable upper limit.

4. Suction control apparatus (101) as claimed in any of claims 1 to 3, further comprising a user-operable over-ride valve (203) operable to bypass said vacuum pressure level regulator (201) and said user-adjustable suction flow rate controller (202).

5. Suction control apparatus (101) as claimed in any of claims 1 to 4, further comprising a flow rate indicator (204) for indicating the suction flow rate within said suction flow path (102).

6. Suction control apparatus (101) as claimed in any of claims 1 to 5, further comprising a vacuum gauge (205) for indicating the vacuum pressure level within said suction flow path (102).

7. Suction control apparatus (101) as claimed in any of claims 1 to 6, housed by a suction control unit (302).

8. Suction control apparatus (101) as claimed in claim 7, wherein said suction control unit (302) is mountable to a trolley (401) for supporting at least one fluid collection canister (106).

9. Suction control apparatus (101) as claimed in any of claims 1 to 8, wherein said pre-set, user non-adjustable maximum regulated suction vacuum level is in the range between a lower limit of equal to or greater than 250 mmHg and an upper limit of less than or equal to 350 mmHg.

10. Suction control apparatus (101) as claimed in claim 3, or any of claims 4 to 9 when dependent upon claim 3, wherein said lower limit is equal to or greater than 10 litres per minute and said upper limit is less than or equal to 100 litres per minute.

11. Suction control apparatus (101) as claimed in any of claims 1 to 10, wherein said outlet (104) is connectable to one of: a hospital central vacuum system, a portable electric suction unit.

12. Suction control apparatus (101) as claimed in any of claims 1 to 11, wherein said vacuum pressure level regulator (201) and said user-adjustable suction flow rate controller (202) are located within said suction flow path (102) between said inlet (103) and said outlet (104) with said vacuum pressure level regulator (201) downstream of said user-adjustable suction flow rate controller (202).

13. Apparatus for use in performing laparoscopy, comprising suction control apparatus (101) as claimed in any of claims 1 to 12.

14. Apparatus for use in performing laparoscopy as claimed in claim 13, wherein said inlet (103) of said suction control apparatus (101) is connected to a suction collection system (105) comprising at least one fluid collection canister (106) and a laparoscopic suction and irrigation set (108).

15. Apparatus for use in performing laparoscopy as claimed in claim 13 or claim 14, wherein said outlet (104) of said suction control apparatus (101) is connected to a suction-generating vacuum source (107).

## Patentansprüche

1. Saugsteuerungsvorrichtung (101) zum Steuern des Saugdurchflusses in laparoskopischer Chirurgie, mit:
einem Saugfließweg (I02) zwischen einem Einlass (I03) und einem Auslass (104),
wobei der Einlass (I03) an ein Saugauffangsystem (105) anschließbar ist, welches mindestens einen Flüssigkeitsauffangkanister (106) umfasst; und
wobei der Auslass (I04) an eine eine Saugwirkung erzeugende Vakuumquelle (107) anschließbar ist, wobei die eine Saugwirkung erzeugende Vakuumquelle (107) nach Anschluss an den Auslass (I04) zur Erzeugung eines Vakuumdruckniveaus der Vakuumquelle betriebsfähig ist, das einen Saugdurchfluss der Vakuumquelle innerhalb des Saugfließweges (I02) herstellt; und
einer durch den Benutzer einstellbaren Saugdurchfluss-Steuerung (202), die zum Steuern des Saugdurchflusses in dem Saugfließweg (I02) betriebsfähig ist; **gekennzeichnet durch**:
einen Vakuumdruckniveauregler (201), der zum Regeln des Vakuumdruckniveaus innerhalb des Saugfließweges (I02) betriebsfähig ist, um zu verhindern, dass ein voreingestelltes, nicht vom Benutzer einstellbares, geregeltes maximales Saugvakuumdruckniveau überschritten wird.

2. Saugsteuerungsvorrichtung (101) nach Anspruch 1, wobei die vom Benutzer einstellbare Saugdurchfluss-Steuerung (202) ein Durchflusssteuerventil umfasst.

3. Saugsteuerungsvorrichtung (101) nach Anspruch 1 oder 2, wobei der Saugdurchfluss in einem Bereich mit einer voreingestellten, vom Benutzer nicht einstellbaren Untergrenze und einer voreingestellten, vom Benutzer nicht einstellbaren Obergrenze einstellbar ist.

4. Saugsteuerungsvorrichtung (101) nach einem der Ansprüche 1 bis 3, weiterhin mit einem vom Benutzer bedienbaren Überbrückungsventil (203), das zur Umgehung des Vakuumdruckniveaureglers (201) und der vom Benutzer einstellbaren Saugdurchfluss-Steuerung (202) betriebsfähig ist.

5. Saugsteuerungsvorrichtung (101) nach einem der Ansprüche 1 bis 4, weiterhin mit einer Durchflussanzeige (204) zum Anzeigen des Saugdurchflusses innerhalb des Saugfließweges (102).

6. Saugsteuerungsvorrichtung (101) nach einem der Ansprüche 1 bis 5, weiterhin mit einem Vakuummesser (205) zum Anzeigen des Vakuumdruckniveaus innerhalb des Saugfließweges (102).

7. Saugsteuerungsvorrichtung (101) nach einem der Ansprüche 1 bis 6, die von einer Saugsteuerungseinheit (302) aufgenommen ist.

8. Saugsteuerungsvorrichtung (101) nach Anspruch 7, wobei die Saugsteuerungseinheit (302) an einem Förderwagen (401) zur Abstützung mindestens eines Flüssigkeitsauffangkanisters (106) anbringbar ist.

9. Saugsteuerungsvorrichtung (101) nach einem der Ansprüche 1 bis 8, wobei das voreingestellte, vom Benutzer nicht einstellbare, maximale geregelte Saugvakuumniveau im Bereich zwischen einer Untergrenze gleich oder größer als 250 mmHg und einer Obergrenze von weniger als oder gleich 350 mmHg liegt.

10. Saugsteuerungsvorrichtung (101) nach Anspruch 3 oder einem der Ansprüche 4 bis 9, wenn diese von Anspruch 3 abhängig sind, wobei die Untergrenze gleich oder größer als 10 Liter pro Minute ist und die Obergrenze weniger als oder gleich 100 Liter pro Minute ist.

11. Saugsteuerungsvorrichtung (101) nach einem der Ansprüche 1 bis 10, wobei der Auslass (I04) an entweder ein zentrales Vakuumsystem der Klinik oder eine tragbare elektrische Saugeinheit anschließbar ist.

12. Saugsteuerungsvorrichtung (101) nach einem der Ansprüche 1 bis 11, wobei sich der Vakuumdruckniveauregler (201) und die vom Benutzer einstellbare Saugdurchfluss-Steuerung (202) in dem Saugfließweg (I02) zwischen dem Einlass (I03) und dem Auslass (I04) befinden, wobei der Vakuumdruckniveauregler (201) der vom Benutzer einstellbaren Durchfluss-Steuerung (202) nachgeschaltet ist.

13. Vorrichtung zur Verwendung bei der Durchführung einer Laparoskopie, mit der Saugsteuerungsvorrichtung (101) nach einem der Ansprüche 1 bis 12.

14. Vorrichtung zur Verwendung bei der Durchführung einer Laparoskopie nach Anspruch 13, wobei der Einlass (I03) der Saugsteuerungsvorrichtung (101) an ein Saugauffangsystem (105) mit mindestens einem Flüssigkeitsauffangkanister (106) und einer laparoskopischen Ansaug- und Spüleinheit (108) angeschlossen ist.

15. Vorrichtung zur Verwendung bei der Durchführung einer Laparoskopie nach Anspruch 13 oder Anspruch 14, wobei der Auslass (I04) der Saugsteuerungsvorrichtung (101) an eine eine Saugwirkung erzeugende Vakuumquelle (107) angeschlossen ist.

## Revendications

1. Appareil de commande d'aspiration (101) destiné à commander le débit d'aspiration au cours d'une opération laparoscopique, comprenant :
un trajet d'écoulement d'aspiration (102) entre une admission (103) et une évacuation (104),
ladite admission (103) pouvant être reliée à un système de collecte d'aspiration (105) comprenant au moins un boîtier de collecte de fluide (106), et
ladite évacuation (104) pouvant être reliée à une source de vide générant une aspiration (107), ladite source de vide générant une aspiration (107), lorsqu'elle est reliée à ladite évacuation (104), servant à générer un niveau de pression à vide de source de vide qui produit un débit d'aspiration de source de vide dans ledit trajet d'écoulement d'aspiration (102) ; et
un organe de commande (202) de débit d'aspiration réglable par l'utilisateur servant à commander le débit d'aspiration dans ledit trajet d'écoulement d'aspiration (102) ; **caractérisé par** :
un régulateur (201) de niveau de pression à vide servant à réguler le niveau de pression à vide dans ledit trajet d'écoulement d'aspiration (102) pour éviter le dépassement d'un niveau prédéfini de pression à vide d'aspiration maximale régulé non réglable par l'utilisateur.

2. Appareil de commande d'aspiration (101) selon la revendication 1, dans lequel ledit organe de commande (202) de débit d'aspiration réglable par l'utilisateur comprend une valve de régulation du débit.

3. Appareil de commande d'aspiration (101) selon la revendication 1 ou la revendication 2, dans lequel ledit débit d'aspiration est réglable dans une plage ayant une limite inférieure prédéfinie non réglable par l'utilisateur et une limite supérieure prédéfinie non réglable par l'utilisateur.

4. Appareil de commande d'aspiration (101) selon l'une quelconque des revendications 1 à 3, comprenant en outre un clapet d'interdiction (203) exploitable par l'utilisateur permettant de dériver ledit régulateur (201) de niveau de pression à vide et ledit organe de commande (202) de débit d'aspiration réglable par l'utilisateur.

5. Appareil de commande d'aspiration (101) selon l'une quelconque des revendications 1 à 4, comprenant en outre un indicateur de débit (204) pour indiquer le débit d'aspiration dans ledit trajet d'écoulement d'aspiration (102).

6. Appareil de commande d'aspiration (101) selon l'une quelconque des revendications 1 à 5, comprenant en outre un vacuomètre (205) pour indiquer le niveau de pression à vide dans ledit trajet d'écoulement d'aspiration (102).

7. Appareil de commande d'aspiration (101) selon l'une quelconque des revendications 1 à 6, logé dans une unité de commande d'aspiration (302).

8. Appareil de commande d'aspiration (101) selon la revendication 7, dans lequel ladite unité de commande d'aspiration (302) peut être montée sur un chariot (401) pour supporter au moins une boîte de collecte de fluide (106).

9. Appareil de commande d'aspiration (101) selon l'une quelconque des revendications 1 à 8, dans lequel ledit niveau prédéfini de vide d'aspiration régulé maximal non réglable par l'utilisateur est dans la plage entre une limite inférieure égale ou supérieure à 250 mmHg et une limite supérieure inférieure ou égale à 350 mmHg.

10. Appareil de commande d'aspiration (101) selon la revendication 3, ou selon l'une quelconque des revendications 4 à 9 lorsqu'il dépend de la revendication 3, dans lequel ladite limite inférieure est égale ou supérieure à 10 litres par minute et ladite limite supérieure est inférieure ou égale à 100 litres par minute.

11. Appareil de commande d'aspiration (101) selon l'une quelconque des revendications 1 à 10, dans lequel ladite évacuation (104) peut être reliée à un parmi : un système de vide central hospitalier, une unité d'aspiration électrique portable.

12. Appareil de commande d'aspiration (101) selon l'une quelconque des revendications 1 à 11, dans lequel ledit régulateur (201) de niveau de pression à vide et ledit organe de commande (202) de débit d'aspiration réglable par l'utilisateur sont situés dans ledit trajet d'écoulement d'aspiration (102) entre ladite admission (103) et ladite évacuation (104), ledit régulateur (201) de niveau de pression à vide étant en aval dudit organe de commande (202) de débit d'aspiration réglable par l'utilisateur.

13. Appareil destiné à être utilisé dans la réalisation d'une laparoscopie, comprenant un appareil de commande d'aspiration (101) selon l'une quelconque des revendications 1 à 12.

14. Appareil destiné à être utilisé dans la réalisation d'une laparoscopie selon la revendication 13, dans lequel ladite admission (103) dudit appareil de commande d'aspiration (101) est reliée à un système de collecte d'aspiration (105) comprenant au moins un boîtier de collecte de fluide (106) et un ensemble laparoscopique d'aspiration et d'irrigation (108).

15. Appareil destiné à être utilisé dans la réalisation d'une laparoscopie selon la revendication 13 ou la revendication 14, dans lequel ladite évacuation (104) dudit appareil de commande d'aspiration (101) est reliée à une source de vide générant une aspiration (107).
